# EUROPEAN PATENT APPLICATION

(11) **EP 2 998 297 A1**
(43) Date of publication of application: **23.03.2016**
(21) Application number: 14185369.7
(22) Date of filing: 18.09.2014
(51) Int. Cl.: C07D 221/14, C07D 401/00, C07D 401/02, C07D 413/02, C07D 417/02, G03F 7/004, G03F 7/039

(54) **Photo-acid generating compounds, compositions comprising said compounds, composite and process for making said composite as well as uses of said compounds**

(71) Applicant: Heraeus Materials Korea Corporation, 153-937 Seoul (KR)
(72) Inventor: Yeongbeom, Lee, Ansan-City, Gyeonggi-do (KR); Hyunyong, Cho, Suwon (KR)
(74) Representative: Herzog, Fiesser & Partner Patentanwälte PartG mbB

(57) **Abstract**

The present invention relates to a compound, wherein the compound comprises at least one structural element A and at least one structural element B, wherein
- structural element A is capable of releasing an acid upon exposure to electromagnetic radiation;
- structural element B comprises at least one basic functional group, wherein the basic functional group comprises a primary, secondary or tertiary amine group.

The present invention also relates to a composition comprising this compound, to a process for producing a composite, to the composite obtained by the process according to the present invention, to a composite comprising a substrate and a coating applied on the surface of the substrate in a patterned structure and to the use of the compound according to the present invention for photoinduced polymerization, photoinduced crosslinking, photoinduced degradation and photoinduced transformation of functional groups.

## Description

The present invention relates to a compound, to a composition comprising this compound, to a process for producing a composite, to the composite obtained by the process according to the present invention, to a composite comprising a substrate and a coating applied on the surface of the substrate in a patterned structure and to the use of the compound according to the present invention for photoinduced polymerization, photoinduced crosslinking, photoinduced degradation and photoinduced transformation of functional groups.

Photoresists are photosensitive films for transfer of images to a substrate. They form negative or positive images. After coating a photoresist on a substrate, the coating is exposed through a patterned photomask to a source of activating energy, such as ultraviolet light, to form a latent image in the photoresist coating. The photomask has areas opaque and transparent to activating radiation that define an image desired to be transferred to the underlying substrate.

Chemical amplification-type photoresists have proven to be useful in achieving high sensitivity in processes for forming ultrafine patterns in the manufacture of semiconductors. These photoresists are prepared by blending a photoacid generator ("PAG") with a polymer matrix having acid labile structures. According to the reaction mechanism of such a photoresist, the photoacid generator generates acid when it is irradiated by the light source, and the main chain or branched chain of the polymer matrix in the exposed or irradiated portion reacts in a so called *"post exposure bake"* (PEB) with the generated acid and is decomposed or cross-linked, so that the polarity of the polymer is considerably altered. This alteration of polarity results in a solubility difference in the developing solution between the irradiated exposed area and the unexposed area, thereby forming a positive or negative image of a mask on the substrate.

It is necessary to prevent acid generated in the exposed area during the course of a photolithography process (i. e. between the exposure with irradiation and the above mentioned *"post exposure bake")* from diffusing or migrating to the unexposed area so as to improve the resolution of the resulting pattern and to obtain a superior profile. In order to prevent the acid diffusion, an alkaline compound has been added to a chemical amplification type photoresist composition as a quencher for the acid in an amount ranging from 5 to 30 % per mole of the photoacid generator. A variety of alkaline compounds have been reported for use in photoresist compositions (see, for example, US-A-2011/0223535, US 7,479,361, US-A-2012/0077120, US-A-2003/0082481, 7,534,554 or US 7,592,126).

However, although the addition of the separately added alkaline compound helps to improve the resolution of the pattern, the activity of the photoacid generator and thus the sensitivity of the photoresist composition towards electromagnetic radiation is decreased. Thus, in the photoresist composition of the prior art the advantageous increase of the resolution of the pattern by means of the addition of a quencher is accompanied by an disadvantageous reduction of the activity of the photoresist composition.

It was therefore an object of the present invention to overcome the disadvantages of the prior art in the field of chemical amplification-type photoresists.

In particular, it was an object of the present invention to provide compounds useful as a photoacid generator in a photoresist composition, wherein a photoresist composition comprising these compounds has a high sensitivity towards electromagnetic radiation, in particular towards electromagnetic radiation with a wavelength in the range of 200 to 500 nm, more particularly towards electromagnetic radiation with a wavelength of 365 nm (i-line), and - at the same time - allows the production of a patterned structure with a higher resolution, compared to the photoresist compositions known from the prior art.

A contribution towards solving these objects is made by a compound, wherein the compound comprises at least one structural element A and at least one structural element B, wherein
- structural element A is capable of releasing an acid upon exposure to electromagnetic radiation;
- structural element B comprises at least one basic functional group, wherein the at least one basic functional group comprises a primary, secondary or tertiary amine group.

The compounds according to the present invention can be used as photoacid generators. Surprisingly, it has been discovered that by combining a structural element that is capable of releasing an acid upon exposure to electromagnetic radiation (which in the photoresist compositions of the prior art was part of the photoacid generator compound) and a structural element that comprises at least one basic functional group (which in the photoresist compositions of the prior art was part of the separately added alkaline compound serving as a quencher) within one compound a photoacid generator is obtained that, when used in a photoacid composition, allows the production of a pattern with a higher resolution compared to a photoresist composition in which the photoacid generating compound and the alkaline compound are added as separate components and - at the same time - is characterized by a sensitivity towards electromagnetic radiation, in particular towards electromagnetic radiation with a wavelength of 200 to 500 nm, more particularly in the range from 300 to 450 nm, more particularly in the range from 350 to 440 nm and even more particularly towards electromagnetic radiation with a wavelength of 365 nm (i-line), that is comparable with the sensitivity of a photoresist composition that is free of any quencher compound.

According to a preferred embodiment of the compound according to the present invention is a non-ionic compound, wherein it is particularly preferred that the compound is selected from the group consisting of
ia) a compound having the general formula (I) 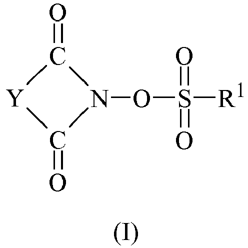 wherein Y and R¹ independently from each other represent an organic group and wherein Y carries the at least one basic functional group, wherein it is preferred that Y represents a methylene group, a divalent, linear or branched aliphatic or heteroaliphatic group having 2-20 carbon atoms or a divalent aromatic or heteroaromatic group having 6-20 carbon atoms, preferably an alkylene group having 2-20 carbon atoms, an aralkylene group having 2-20 carbon atoms, a fluoromethylene group, a linear or branched fluorinated alkylene group having 2-20 carbon atoms, a cyclohexylene group, a phenylene group, a substituted or unsubstituted divalent group possessing a norbornene skeleton, or a group wherein these groups are substituted with an aryl group having six or more carbon atoms or an alkoxyl group having one or more carbon atoms;
ib) a compound having the general formula (II) 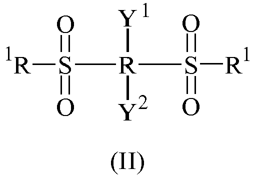 wherein Y¹, Y², R, R¹ and R² independently from each other represent an organic group and wherein at least one of Y¹ and Y² carries the at least one basic functional group, wherein R preferably represents a methylene group, an aliphatic or heteroaliphatic group or an aromatic group or a heteroaromatic group or a combination thereof and wherein it is preferred that Y¹ and Y² individually represent an aryl group, a hydrogen atom, a linear or branched monovalent aliphatic hydrocarbon group, a cycloalkyl group, an aralkyl group, or another monovalent organic group having a hetero atom, provided that at least one of Y¹ and Y² represents an aryl group, or Y¹ and Y² are bond to form a monocyclic or polycyclic ring having at least one unsaturated bond, or Y¹ and Y² are bond to form a group shown by the following formula (II') 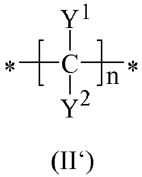 wherein Y¹ and Y² individually represent a hydrogen atom, a halogen atom, a linear or branched aliphatic or heteroaliphatic group, in particular an alkyl group or a cycloalkyl group, or an aromatic or heteroaromatic group, in particular an aryl group or an aralkyl group, or Y¹ and Y², each bonded to the same or different carbon atoms, may form a monocarboxylic structure, and n is an integer from 2 to 10;
   or
ic) a compound having the general formula (IIIa) or (IIIb) 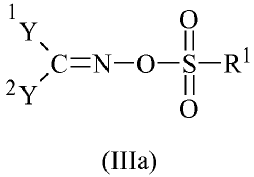 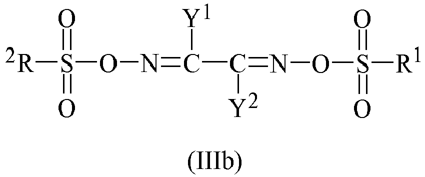 wherein Y¹, Y², R¹ and R² independently from each other represent an organic group and wherein at least one of Y¹ and Y² carries the at least one basic functional group, wherein in formula (IIIa) Y¹ preferably represents a -CN group and Y² represents a phenyl group, a tosyl group, a 1-naphthyl group, a trifluoromethyl group or a nonafluoro-n-butyl group and wherein in formula (IIIb) Y¹ and Y² independently from each other represent a phenyl group, a tosyl group, a 1-naphthyl group, a trifluoromethyl group or a nonafluoro-n-butyl group.

In the compounds having the general formula (I), (II), (IIIa) or (IIIb) R¹ preferably is selected from the group consisting of
- a linear, branched, or cyclic aliphatic group having a carbon number of 1 to 18, which may be substituted by one or more halogen atom(s);
- a linear, branched, or cyclic aliphatic group having a carbon number of 3 to 18 which comprises least one moiety selected from the group consisting of-O-, -S-, -C(=O)-O-, -C(=O)-S-, -O-S(=O)₂-, -O-C(=O)-O-, -C(=O)-NH-, -O-C(=O)-NH-, -C(=O)-NRₐ-, -O-C(=O)-NRₐ-, and -C(=O)-NRₐR_{b}, wherein Rₐ and R_{b} are each independently a linear, branched, or cyclic aliphatic group with a carbon number of 1 to 10, which may be the same or different and may be connected to form an alicyclic group, and wherein the linear, branched, or cyclic aliphatic group optionally comprises at least one halogen atom;
- an aryl or heteroaryl group having a carbon number of 4 to 18 in which one or more hydrogen atoms may be substituted by a halogen atom, an aliphatic, an haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group; and
- an arylalkyl or heteroarylalkyl group having a carbon number of 4 to 18 in which one or more hydrogen atoms in the aryl or heteroaryl group may be substituted by a halogen atom, an aliphatic, a haloalkyl, an alkoxy, a haloalkoxy, an alkylthio, a bisalkylamino, an acyloxy, an acylthio, an acylamino, an alkoxycarbonyl, an alkylsulfonyl, an alkylsulfinyl, an alicyclic, a heterocyclic, an aryl, an alkylaryl, a cyano, or a nitro group.

Preferably, R¹ is selected from the group consisting of an optionally substituted, linear or branched alkyl group having 1-18 carbon atoms, an optionally substituted cycloalkyl group having 5-18 carbon atoms, a linear or branched fluorinated of perfluorinated alkyl group having 1-18 carbon atoms, a monovalent hydrocarbon group possessing a bicyclo ring having 7-15 carbon atoms, or an aryl group having 6-12 carbon atoms.

Suitable examples of residues R¹ are all the groups that are bond to the nitrogen atom of the imide group in compounds 1 to 78 on pages 7 to 14 in US 2012/0289697 A1. The disclosure of US 2012/0289697 A1 with respect to the organic residues "R⁰⁷" that can be bond to the sulfur atom in the functional group -O-S(O₂)-R in the structural formula (I) of US 2012/0289697 A1 is incorporated herein by reference.

According to a particularly preferred embodiment of the compound according to the present invention R¹ represents a linear, branched or cyclic perfluoroalkyl group having the general formula -CₓF_{y}, in which x is an integer in the range from 1-8 and y is an integer in the range from 3-17. Examples of such residues R¹ are -C₆F₅ (pentafluorophenyl), -C₇F₇, -CF₃, -C₂F₅,-C₃F₇ and -C₄F₉.

The at least one basic functional group of the compound according to the present invention, that in structural formula (I), (II), (IIIa) and (IIIb) is bond to groups Y¹ and/or Y², preferably has a pK_{A}-value determined at 25°C in the range from 2.0 to 25.0, more preferably in the range from 5.0 to 14.0 and most preferred in the range from 7.0 to 12.0.

It is furthermore preferred that in the compound according to the present invention the at least one basic functional group has the general formula (IVa) or (IVb) 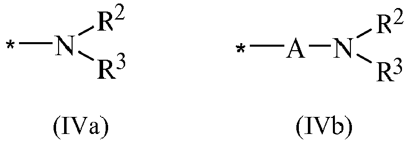 in which "*" indicates the bond to the compound, in particular to groups Y¹ and/or Y², and wherein
- R² and R³ independently from each other represent
   -- a hydrogen atom,
   -- a linear or branched, optionally substituted aliphatic or heteroaliphatic group having 1-20 carbon atoms, in particular an alkyl group having 1-20 carbon atoms, preferably 1-10 carbon atoms and most preferably 1-5 carbon atoms,an optionally substituted cycloalkyl group having 5-20 carbon atoms, preferably 5-15 carbon atoms and most preferably 5-10 carbon atoms, or a linear or branched, optionally substituted alkyl carbonyl group having 2-20 carbon atoms, preferably 2-15 carbon atoms and most preferably 2-10 carbon atoms, or
   -- an aromatic or heteroaromatic group having 6-20 carbon atoms, in particular an optionally substituted aryl group having 6-20 carbon atoms, preferably 6-15 carbon atoms and most preferably 6-10 carbon atoms,
      or
- R² and R³ together form a heterocyclic group that, in addition to the nitrogen atom shown in the general formula (IV), optionally comprises a further heteroatom selected from the group consisiting ofN, O and S;
- A represents
   -- an optionally substituted divalent aliphatic or heteroaliphatic group having 1-20 carbon atoms, in particular a C₁-C₂₀ alkylene group, preferably an optionally substituted C₁-C₁₀ alkylene group and most preferably an optionally substituted C₁-C₅ alkylene group, an optionally substituted C₁-C₂₀ oxyalkylene group, preferably an optionally substituted C₁-C₁₀ oxyalkylene group and most preferably an optionally substituted C₁-C₅ oxyalkylene group, a carbonyl group, a C₂-C₂₀ alkylene carbonyl group, preferably C₂-C₁₀ alkylene carbonyl an most preferably a C₂-C₅ alkylene carbonyl, or a group selected from the group consisting of *-S- (CH₂)ₙ- and *-S-(CH₂)ₙ-CO-, in which n is an integer from 1 to 20, preferably a *-S- (CH₂)₂-group or a *-S- (CH₂)₂CO- group, or
   -- a divalent aromatic or heteroaromatic group having 6-20 carbon atoms.

Particularly preferred basic functional groups have a general formula selected from the group consisting of formulae (Va) to (Vp) (the hydrogen atoms in the rings of formula (Ve) to (Vp) are not shown for clarity): 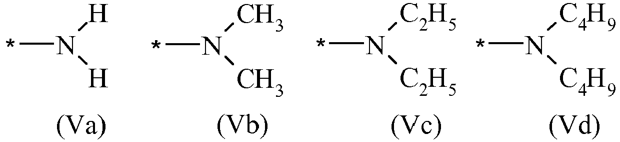 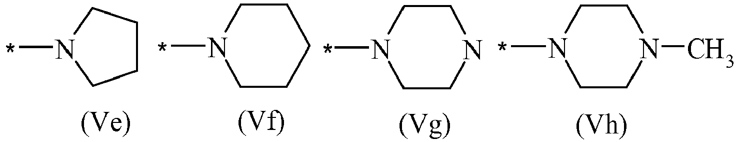 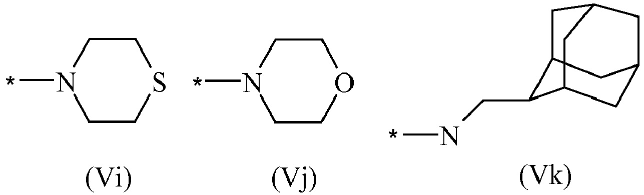 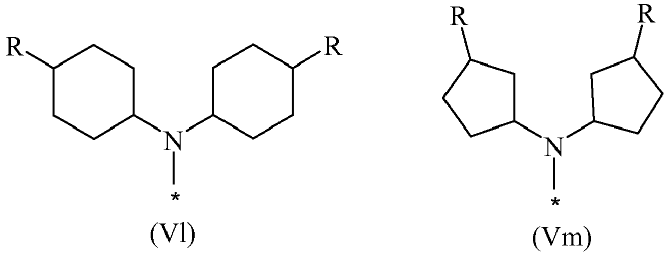 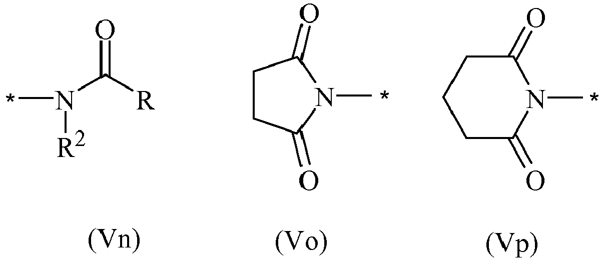 wherein R² is as defined in connection with the general formula (IVa) and (IVb) and R represents a hydrogen or an aliphatic group having 1-20 carbon atoms.

An example of a suitable compound having the general formula (II) is a compound selected from the group consisting of (IIa) and (IIb) 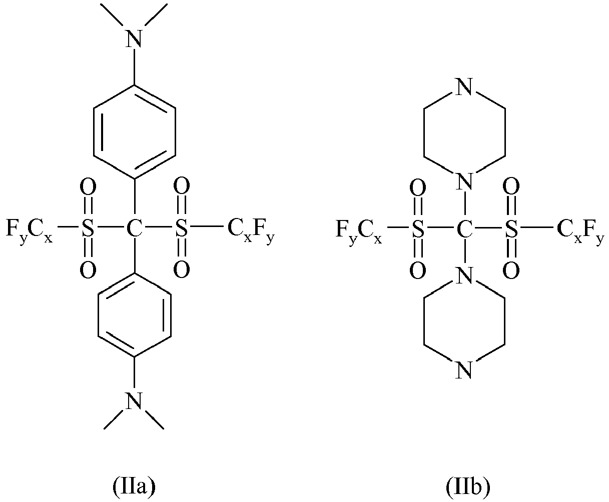 an example of a suitable compound having the general formula (IIIa) is a compound selected from the group consisting of (IIIa-1) and (IIIa-2) 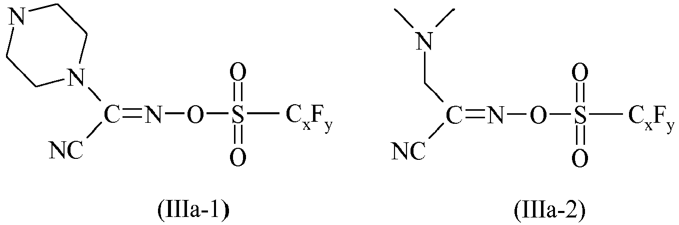 and an example of a suitable compound having the general formula (IIIb) is a compound selected from the group consisting of (IIIb-1) and (IIIb-2) 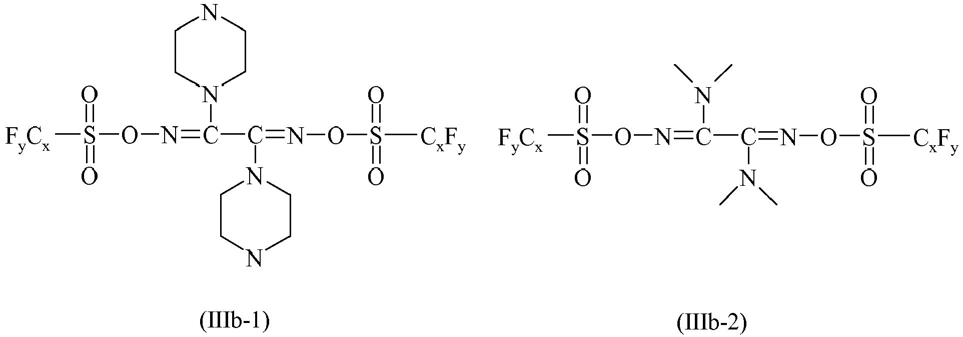 wherein hydrogens atoms in formulae (IIa), (IIb) (IIIa-1), (IIIa-2), (IIIb-1) and (IIIb-2) are not shown for clarity and wherein x and y are defined as above.

According to a particularly preferred embodiment of the compound according to the present invention the compound is a sulfoimide falling under the general formula (I) as described above, in particular a compound having the general formula (Ia) 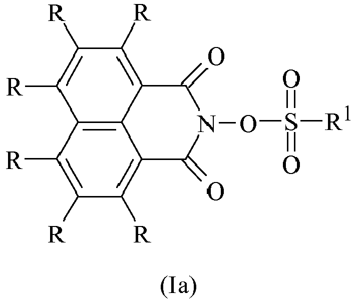 wherein R¹ is as defined before and wherein R independently from each other represents a hydrogen, a halogen, a hydroxyl group, a thiol group or an aliphatic or heteroaliphatic group with 1-20 carbon atoms, in particular an ether group, a thioether group or a C₁-C₂₀ alkyl group, with the provisio that at least one of groups R represents a basic group -NR²R³ or -A-NR²R³ as defined above. According to a particular embodiment of the compound according to the present invention, at least two groups R or at least three groups R represent a basic group-NR²R³ or -A-NR²R³ as defined above.

In this context it is particularly preferred that the compound according to the present invention has the general formula (Ia-1), (Ia-2), (Ia-3) or (Ia-4): 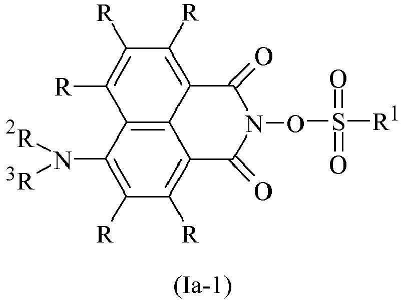 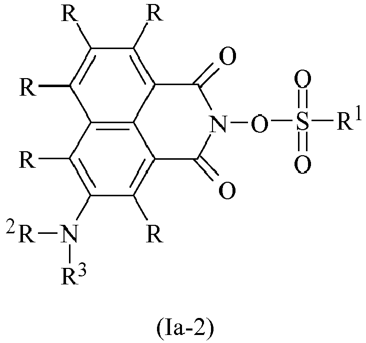 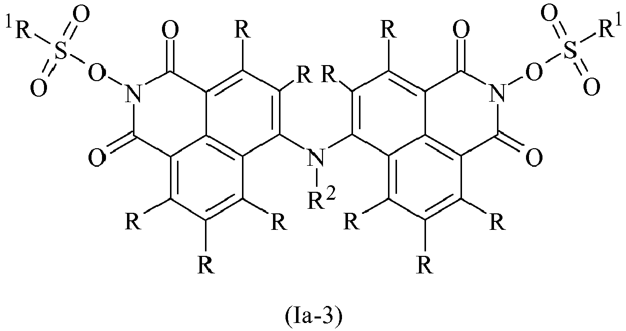 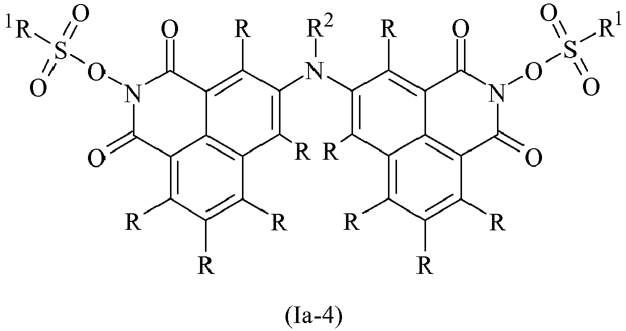 wherein R, R², R³ and group -NR²R³ are as defined above.

Examples of compounds having the general formula (Ia-1) or (Ia-3) can be selected from the group consisting of formulae (Ia-a) to (Ia-r) (hydrogen atoms are not shown for clarity):

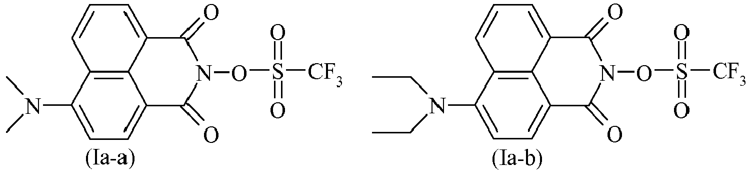

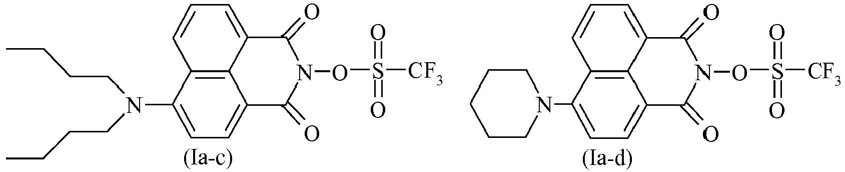

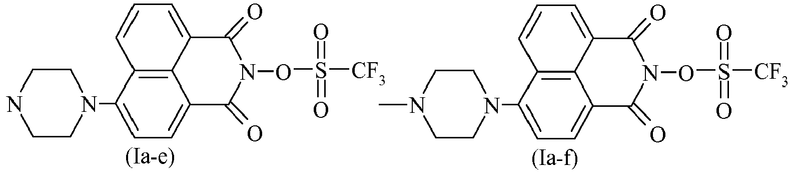

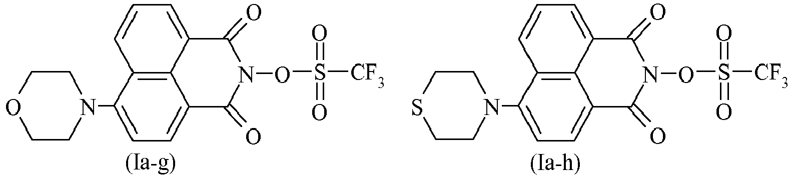

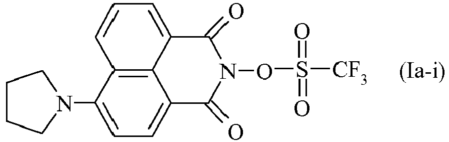

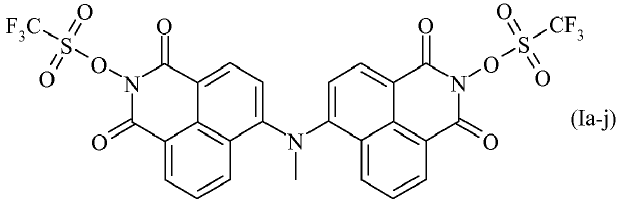

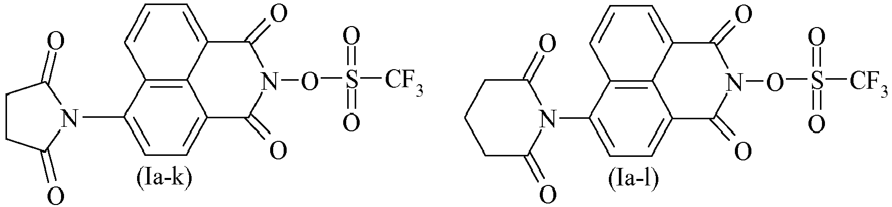

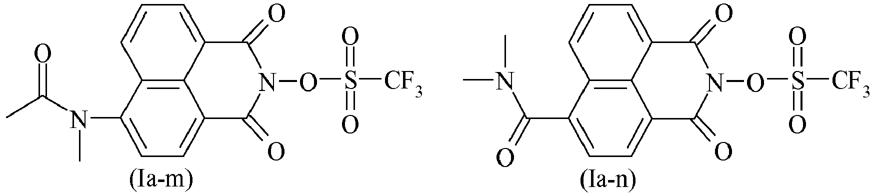

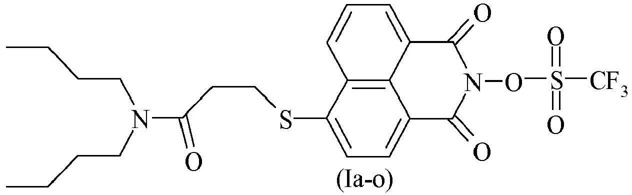

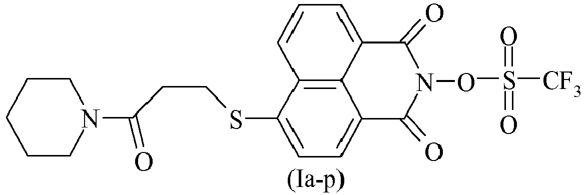

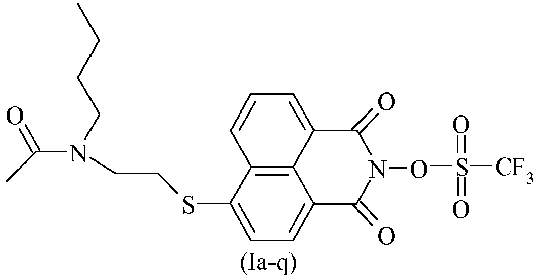

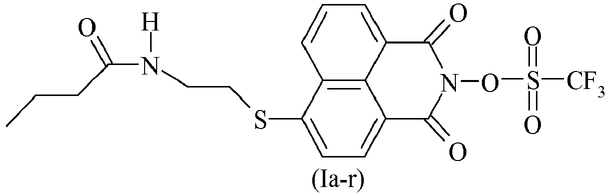

Also suitable are the corresponding compounds of the general formula (Ia-2) and (Ia-4) in which the basic functional group -NR²R³ is bond to the aromatic ring system at position 3 (instead of position 4 as shown in formulae (Ia-a) to (Ia-k)).

Compounds of the general formula (Ia-1) and (Ia-2) can be prepared by reacting 3-substituted anhydrides such as 4-bromo-1,8-napthalic anhydride, 3-bromo-1,8-naphatlic anhydride, 4-hydroxy-1,8-napthalic anhydride or 3-hydroxy-1,8-naphatlic anhydride with the corresponding amine HNR₂R₃), followed by a reaction with hydroxylamine to form the N-hydroxyimide. In a final process step the thus obtained N-hydroxyimide is reacted with sulfonyl halogenates such as sulfonyl chlorides (R¹-S(O₂)-Cl) as shown in the following reaction scheme):

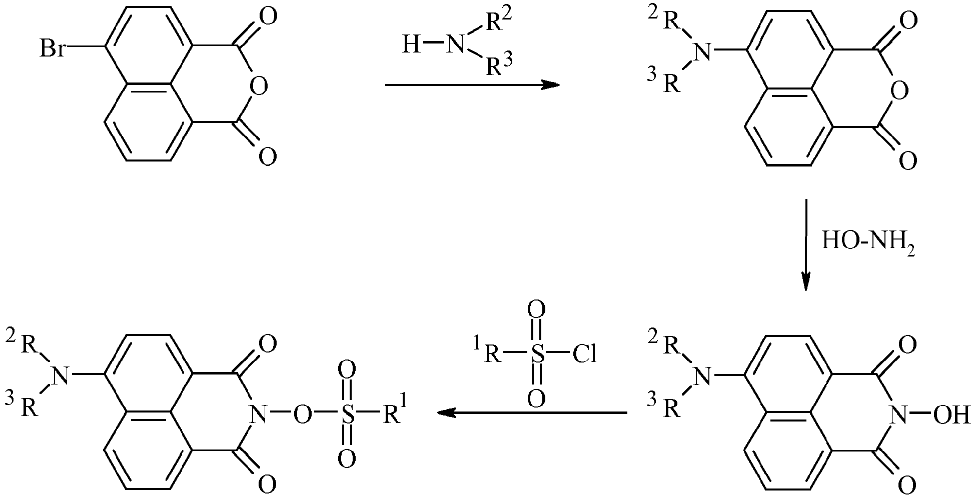

Further examples of a compound according to the present invention having the structural formula (Ia) and comprising more than one basic functional group is shown in the following formula (Ia-s), in which R⁴ and R⁵ are as defined for R² and R³, X and Y are as defined for A and n is an integer in the range from 1 to 20: 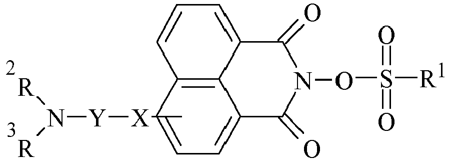 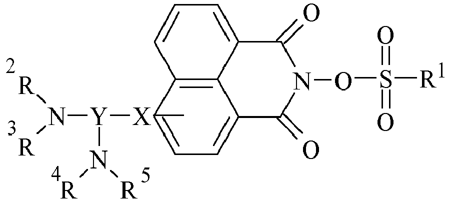 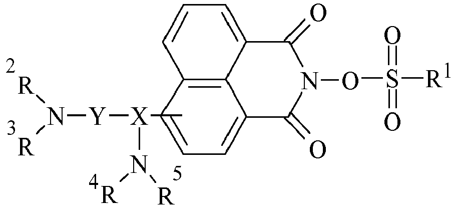 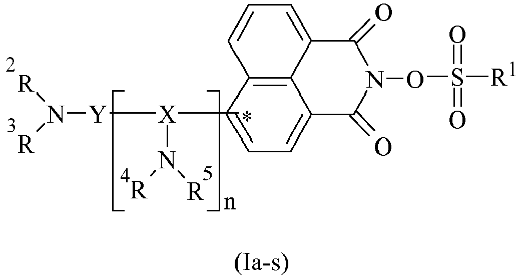 A contribution towards solving the objects of the present invention is also made by a composition comprising
(i) at least one compound according to the present invention;
(ii) at least one compound capable of being imparted with an altered solubility in an aqueous solution, preferably an aqueous base solution, in the presence of an acid;
(iii) at least one organic solvent;
(iv) optionally at least one additive being different from components (i), (ii) and (iii).

The composition according to the present invention can be used as a photoresist in a variety of applications, in particular for the production of electronic devices, including flat panel display (in this case the photoresist can be coated glass substrate or a layer of indium tin oxide) and a semiconductor device (in this case the photoresist can be coated onto a silicon wafer substrate). Various exposure radiations can be used, including an exposure with electromagnetic radiation having a wavelength in the range of 200 to 500 nm, more particularly in the range from 300 to 450 nm, more particularly in the range from 350 to 440 nm and even more particularly electromagnetic radiation having a wavelength of 365 nm (i-line), 435 nm (g-line) or 405 nm (h-line), wherein an electromagnetic radiation with a wavelength of 365 nm is most preferred.

The composition according to the present invention comprises as component (ii) one or more photoresist polymers or copolymers, which may be base soluble or insoluble. The photoresist composition according to the present invention may be a positive tone or negative tone composition. In the case of a positive tone composition the solubility of component (ii) is increased upon reaction with the acid released from the compound according to the present invention. In this case, photoresist polymers or copolymers with acid labile groups are used as component (ii) which are insoluble in aqueous base solution, but which in the presence of the acid are catalytically deprotected such that they become soluble in an aqueous base solution. In the case of a negative tone composition, the solubility of component (ii) is decreased upon reaction with the acid released from the compound according to the present invention. In this case, photoresist polymers or copolymers are used as component (ii) which are soluble in aqueous base solution), but which in the presence of the acid are crosslinked such that they become insoluble in an aqueous base solution.

Examples of photoresist polymers that may be used as component (ii) in a positive tone composition include without limitation, aromatic polymers, such as homopolymers or copolymers of hydroxystyrene protected with an acid labile group; acrylates, such as for example, poly(meth)acrylates with at least one unit containing a pendant alicyclic group, and with the acid labile group being pendant from the polymer backbone and/or from the aclicyclic group, cycloolefin polymers, cycloolefin maleic anhydride copolymers, cycloolefin vinyl ether copolymers, siloxanes; silsesquioxanes, carbosilanes; and oligomers, including polyhedral oligomeric silsesquioxanes, carbohydrates, and other cage compounds. The foregoing polymers or oligomers are appropriately functionalized with aqueous base soluble groups, acid-labile groups, polar functionalities, and silicon containing groups as needed.

Examples of copolymers that may be used as component (ii) in the positive tone compositions of the present invention include without limitation poly(p-hydroxystyrene)-methyl adamantyl methacrylate (PHS-MAdMA), poly(p-hydroxystyrene)-2-ethyl-2-adamantyl methacrylate (PHS-EAdMA), poly(p-hydroxystyrene)-2-ethyl-2-cyclopentyl methacrylate (PHS-ECpMA), poly(p-hydroxy-styrene)-2-methyl-2-cyclopentyl methacrylate (PHS-MCpMA) or PHS-EVE.

Preferably, the at least one component (ii) in a positive tone composition is a poly(hydroxystyrene)-resin in which at least a part of the hydroxy groups is substituted by protective groups. Preferred protective groups are selected from the group consisting of a tert-butoxycarbonyloxy group, a tert-butyloxy group, a tert-amyloxycarbonyloxy group and an acetal group. Furthermore suitable as component ii) are all the polymers and copolymers which in paragraphs [0068] to [0114] of EP 1 586 570 A1 are described as *"acid-dissociable group-containing resins".* The disclosure of EP 1 586 570 A1 with respect to these resins is enclosed herein by reference a forms a part of the disclosure of the present invention.

Preferred negative tone compositions comprise a mixture of materials that will cure, crosslink or harden upon exposure to acid. Preferred negative acting compositions comprise, as component (ii), a polymer binder such as a phenolic or non-aromatic polymer, a crosslinker component as an additive (iv) and the component according to the present invention as component (i). Suitable polymer binders and crosslinkers for such negative tone photoresist compostions and the use thereof have been disclosed in EP-A-0 164 248 and US 5,128,232. Preferred phenolic polymers for use as component (ii) include novolaks and poly(vinylphenol)s. Novolak resins are the thermoplastic condensation products of a phenol and an aldehyde. Examples of suitable phenols for condensation with an aldehyde, especially formaldehyde, for the formation of novolak resins include phenol, m-cresol, o-cresol, p-cresol, 2,4-xylenol, 2,5-xylenol, 3,4-xylenol, 3,5-xylenol and thymol. An acid catalyzed condensation reaction results in the formation of a suitable novolak resin which may vary in molecular weight from about 500 to 100,000 daltons. Polyvinyl phenol resins are thermoplastic polymers that may be formed by block polymerization, emulsion polymerization or solution polymerization of the corresponding monomers in the presence of a cationic catalyst. Vinylphenols useful for the production of polyvinyl phenol resins may be prepared, for example, by hydrolysis of commercially available coumarin or substituted coumarins, followed by decarboxylation of the resulting hydroxy cinnamic acids. Useful vinylphenols may also be prepared by dehydration of the corresponding hydroxy alkyl phenols or by decarboxylation of hydroxy cinnamic acids resulting from the reaction of substituted or nonsubstituted hydroxybenzaldehydes with malonic acid. Preferred polyvinyl phenol resins prepared from such vinylphenols have a molecular weight range of from about 2,000 to about 60,000 daltons. Preferred crosslinkers for use as component (iv) include amine-based materials, including melamine, glycolurils, benzoguanamine-based materials and urea-based materials. Melamine-formaldehyde polymers are often particularly suitable. Such crosslinkers are commercially available, e.g. the melamine polymers, glycoluril polymers, urea-based polymer and benzoguanamine polymers, such as those sold by Cytec under tradenames Cymel 301, 303, 1170, 1171, 1172, 1123 and 1125 and Beetle 60, 65 and 80.

As component (iii) the composition according to the present invention comprises at least one organic solvent.

The organic solvent may be any solvent capable of dissolving the component (ii) and the component (i) to generate a uniform solution, and one or more solvents selected from known materials used as the solvents for conventional chemically amplified resists can be used. Specific examples of the organic solvent include ketones such as acetone, methyl ethyl ketone, cyclohexanone, methyl isoamyl ketone and 2-heptanone, polyhydric alcohols and derivatives thereof such as ethylene glycol, ethylene glycol monoacetate, diethylene glycol, diethylene glycol monoacetate, propylene glycol, propylene glycol monoacetate, dipropylene glycol, or the monomethyl ether, monoethyl ether, monopropyl ether, monobutyl ether or monophenyl ether of dipropylene glycol monoacetate, cyclic ethers such as dioxane, and esters such as methyl lactate, ethyl lactate (EL), methyl acetate, ethyl acetate, butyl acetate, methyl pyruvate, ethyl pyruvate, methyl methoxypropionate, and ethyl ethoxypropionate. These organic solvents can be used alone, or as a mixed solvent containing two or more different solvents. Particularly preferred organic solvents (iii) are selected from the group consisting of a ketone, an ether and ester.

Furthermore, the composition according to the present invention may also comprise at least one additive being different from components (i), (ii) and (iii). Suitable additives include surfactants for improving the ease of application, dissolution inhibitors, plasticizers, stabilizers, colorants, halation prevention agents and - in case of negative tone compositions - the above mentioned crosslinkers.

According to a preferred embodiment of the composition according to the present invention the composition comprises
(i) 0.05 to 15 wt.-%, preferably 0.1 to 12.5 wt.-% and most preferably 1 to 10 wt.-% of the compound according to the present invention;
(ii) 5 to 50 wt.-%, preferably 7.5 to 45 wt.-% and most preferably 10 to 40 wt.-% of the compound capable of being imparted with an increased solubility in an aqueous solution in the presence of an acid and
(iv) 0 to 10 wt.-%, preferably 0.01 to 7.5 wt.-% and most preferably 0.1 to 5 wt.-% of the further additive;
wherein the reminder in the composition is the organic solvent (iii).

As in the compounds according to the present invention the functional basic group serving as a quencher for the acid group that is released upon exposure to electromagnetic radiation is a part of the photoacid generator compound, it is not necessary to add a separate basic component as a quencher (as it is necessary in the photoresist compositions known from the prior art). According to a preferred embodiment of the composition according to the present invention this composition preferably comprises less than 5 wt.-%, more preferably less than 1 wt.-%, even more preferably less than 0.1 wt.% and most preferably 0 wt.-% of a basic compound being different from components (i) through (iv), such as hydroxides, carboxylates, amines, imines, and amides.

A contribution towards solving the objects of the present invention is also made by a process for producing a composite comprising a substrate and a coating that is applied onto the substrate in a patterned structure, the process comprising the steps of
(α1) applying a layer of the composition according to the present invention onto the surface of the substrate and at least partial removal of the organic solvent (iii);
(α2) exposing selected areas of the layer to electromagnetic radiation, thereby releasing an acid from the compound (i) in the areas exposed to the electromagnetic radiation;
(α3) optionally heating the layer to impart compound (ii) in the areas in which the acid has been released with an altered solubility in an aqueous solution;
(α4) at least partial removal of the layer.

In process step (α1) layer of the composition according to the present invention onto the surface of the substrate and at least partial removal of the organic solvent (iii).

Substrates may be any dimension and shape, and are preferably those useful for photolithography, such as silicon, silicon dioxide, silicon-on-insulator (SOI), strained silicon, gallium arsenide, coated substrates including those coated with silicon nitride, silicon oxynitride, titanium nitride, tantalum nitride, ultrathin gate oxides such as hafnium oxide, metal or metal coated substrates including those coated with titanium, tantalum, copper, aluminum, tungsten, alloys thereof, and combinations thereof. Preferably, the surfaces of substrates herein include critical dimension layers to be patterned including, for example, one or more gate-level layers or other critical dimension layer on the substrates for semiconductor manufacture. Such substrates may preferably include silicon, SOI, strained silicon, and other such substrate materials, formed as circular wafers having dimensions such as, for example, 20 cm, 30 cm, or larger in diameter, or other dimensions useful for wafer fabrication production.

Application of the composition according to the present invention onto the substrate may be accomplished by any suitable method, including spin coating, spray coating, dip coating, doctor blading, or the like. Applying the layer of photoresist is preferably accomplished by spin-coating the photoresist using a coating track, in which the photoresist is dispensed on a spinning wafer. During dispense, the wafer may be spun at a speed of up to 4,000 rpm, preferably from about 500 to 3,000 rpm, and more preferably 1,000 to 2,500 rpm. The coated wafer is spun to remove the organic solvent (iii), and baked on a hot plate to remove residual solvent and free volume from the film to make it uniformly dense.

In process step (α2) selected areas of the layer are exposed to electromagnetic radiation, thereby releasing an acid from the compound (i) in the areas exposed to the electromagnetic radiation. As stated above, various exposure radiations can be used, including an exposure with electromagnetic radiation having a wavelength of 365 nm (i-line), 435 nm (g-line) or 405 nm (h-line), wherein electromagnetic radiation having a wavelength of 365 nm is particularly preferred.

Such a pattern-wise exposure can be carried out using an exposure tool such as a stepper, in which the film is irradiated through a pattern mask and thereby is exposed pattern-wise. The method preferably uses advanced exposure tools generating activating radiation at wavelengths capable of high resolution including extreme-ultraviolet (EUV) or e-beam radiation. It will be appreciated that exposure using the activating radiation decomposes the component according to the present invention that is contained in the photoresist layer in the exposed areas and generates acid and decomposition by-products, and that the acid then effects a chemical change in the polymer compound (ii) (deblocking the acid sensitive group to generate a base-soluble group, or alternatively, catalyzing a cross-linking reaction in the exposed areas). The resolution of such exposure tools may be less than 30 nm.

In process step (α3) the layer can optionally be heated to impart compound (ii) in the areas in which the acid has been released with an altered solubility in an aqueous solution. In this so called "*post*-*exposure bake"* the solubility differences between exposed and unexposed regions of the coating layer are created or enhanced. Typically post-exposure bake conditions include temperatures of about 50°C or greater, more specifically a temperature in the range of from about 50°C to about 160°C for 10 seconds to 30 minutes, preferably for 30 to 200 seconds. According to a particular embodiment of the process according to the present invention no heat treatment is performed after process step (α2) and before (α4).

In process step (α4) the layer is at least partially removed with an aqueous solution, preferably an aqueous base solution. This can be accomplished by treating the exposed photoresist layer with a suitable developer capable of selectively removing the exposed portions of the film (where the photoresist is positive tone) or removing the unexposed portions of the film (where the photoresist is negative tone). Preferably, the photoresist is positive tone based on a polymer having acid sensitive (deprotectable) groups, and the developer is preferably a metal-ion free tetraalkylammonium hydroxide solution.

A contribution towards solving the objects of the present invention is also made by a composite obtained by the process according to the present invention. This composite is characterised in that it comprises a substrate and a coating applied on the surface of the substrate in a patterned structure, wherein the coating comprises a compound according to the present invention.

A contribution towards solving the objects of the present invention is also made by a composite comprising a substrate and a coating applied on the surface of the substrate in a patterned structure, wherein the coating comprises a compound according to the present invention.

A contribution towards solving the objects of the present invention is also made by the use of the compound according to the present invention for photoinduced polymerization, photoinduced crosslinking, photoinduced degradation and photoinduced transformation of functional groups. The compound according to the present invention is in particular suitable for use in in protective coatings, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufacturing.

The invention is now explained in more detail with the aid of non-limiting figures and examples.

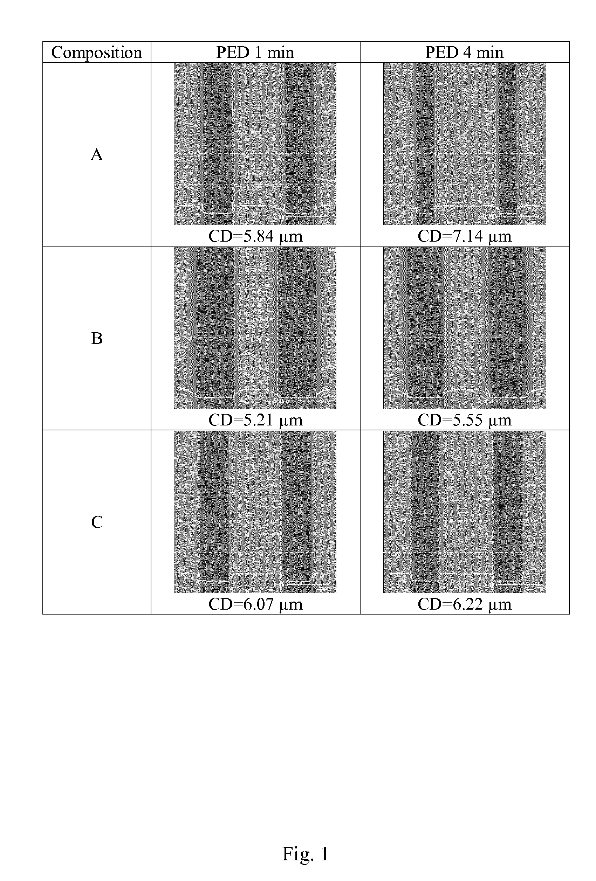

Figure 1 is a comparison between conventional photoresist composition comprising NIT (N-trifluoromethylsulfonyl-oxy-1,8-naphthalimide) as the photoacid generator and a photoresist composition according to the present invention comprising the compound with the general formula (Ia-d) as the photoacid generator, wherein layers of the composition have been exposed to i-line radiation in a patterned structure.

### EXAMPLES

### Example 1:

### Preparation of compound (Ia-d)

All the compounds and the solvents were of experimental grade. 1H, 13C and 19F-NMR spectra were measured on a Bruker AV-400 spectrometer with chemical shifts reported as ppm (in CDCl₃/DMSO-d6, TMS as internal standard). Mass spectra were measured on a Shimadzu GCMS-QP2010SE spectrometer. Melting points were determined by an Electrothermal-melting point apparatus and are uncorrected. UV-vis. absorption spectra were determined on a DU 800 UV-vis spectrometer.

### Preparation of 6-Piperidino-2-oxa-1,3-phenalenedione

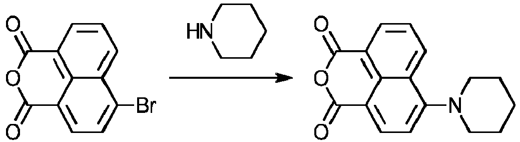

A mixture of 4-bromo-1,8-naphthalic anhydride (2.77 g, 10.0 mmol), and piperidine (1.97 ml, 20 mmol) in 2-methoxyethanol (5 ml) was refluxed for 16 h, then the reaction mixture was cooled to room temp to obtain a yellow solid, which was purified by recrystallization from ethanol to give 2.41 g (86%) of 6-Piperidino-2-oxa-1,3-phenalenedione in the form of orange needles. CAS: 87223-12-9. M.p. 170-172°C (lit. 175-176°C) 1H NMR (400 MHz, CDCl₃) δ: 8.54 (d, 1H), 8.46 (d, 1H), 8.41 (d, 1H), 7.68 (t, 1H), 7.17 (d, 1H), 3.29 (s, 4H), 1.90 (s, 4H), 1.75 (s, 2H); 13C NMR (100 MHz, CDCl₃) δ: 161.5, 160.7, 158.4, 135.0, 133.1, 132.4, 132.2, 126.1, 125.6, 119.2, 114.9, 110.7, 54.3, 26.1, 24.2; MS m/z: 281(M+, 83), 280 ([M-1]+, 100).

### Preparation of 2-Hydroxy-6-piperidino-2-aza-2H-phenalene-1,3-dione

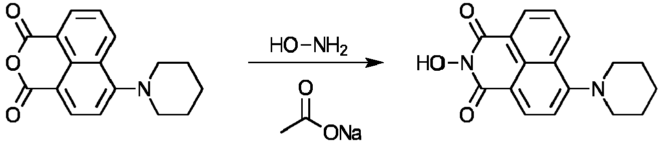

To a clean, dry 20 mL reaction vial equipped on magnetic stirring hot plate with thermosensor was added deionized water (10 mL), sodium acetate (0.3324 g, 4.0525 mmol), hydroxylamine hydrochloride (0.2915 g, 4.1947 mmol), and 4-piperidinyl-1,8-naphthalic anhydride (1 g, 3.5548 mmol). The reaction mixture was heated to 75-80°C and hold for 16.5 hrs. The reaction was then cooled to < 30°C and the mixture were filtered. The solids were slurred in deionized water (15 mL) and refiltered and washed with more deionized water. The solid was air-dried for 12 hours, and then washed with methanol (15 mL) twice. The solid was filtered and air-dried 12 hours, then dried in vacuo at 60°C for 24 hrs. 2.41 g (86 %) of the product obtained in the form of an orange powder. 1H NMR (400 MHz, ppm, DMSO-d6) δ: 10.54 (s, 1H), 8.43 (d, 1H), 8.33 (d, 2H), 7.77 (t, 1H), 7.23 (t, 1H), 3.17 (s, 4H), 1.78 (s, 4H), 1.62 (s, 2H).

Preparation of 6-Piperidino-2-(trifluoromethylsulfonyloxy)-2-aza-2H-phenalene-1,3-dione

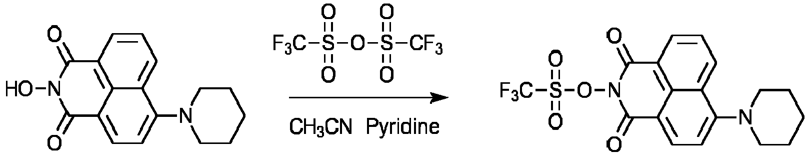

To a clean, dry 20 mL vial equipped with thermosensor, additional syringe, magnetic stirring, nitrogen balloon on cooling bath; was charged acetonitrile (10 mL), 2-Hydroxy-6-piperidino-2-aza-2H-phenalene-1,3-dione (1 g, 3.375 mmol), pyridine (0.41 mL, 5.062 mmol). The mixture was cooled to < 10°C and stirred. Triflic anhydride (0.6 mL, 5.062 mmol) was charged to the syringe and a slow addition was started. The reaction is held below 15°C during the addition. After the addition, the reaction is warmed to room temperature overnight. The reaction is then heated to 75-80°C until a clear solution is observed. The reaction is then cooled to < 10°C. The crude solid is then filtered washed with methanol and air-dried 12 hours, then dried in vacuo at 40°C for 48 hrs. The product is yellowish orange and was obtained in an amount of 0.90g (62.3 %). 1H NMR (400 MHz, CDCl₃) δ: 8.60 (dd, 1H), 8.52 (d, 1H), 8.45 (dd, 1H), 7.72 (t, 1H), 7.20 (d, 1H), 3.29 (t, 4H), 1.90 (q, 4H), 1.75 (q, 2H), 19F NMR (400 MHz, CDCl₃) δ: -70.65 (s, CF3), UV-vis (nm) λmax=420 nm (0.001 % in PGMEA)

### Example 2:

### Preparation of photoresist compositions

The following photoresist compositions have been prepared:
**Composition A** (not according to the present invention)
   - 0.449 g NIT (N-trifluoromethylsulfonyl-oxy-1,8-naphthalimide)
   - 15 g PHS-EVE (a tri-block copolymer obtained by living cationic polymerization of p-hydroxystyrene (PHS) and ethyl vinyl ether (EVE) with 30 % EVE
   - 85 g of PGMEA as solvent
**Composition B** (not according to the present invention)
   - 0.449 g of NIT
   - 15 g PHS-EVE (30 % EVE)
   - 85 g of PGMEA as solvent
   - 0.026 g of triethylamine as quencher
**Composition C** (according to the present invention)
   - 0.557 g 6-Piperidino-2-(trifluoromethylsulfonyloxy)-2-aza-2H-phenalene-1,3-dione obtained in Example 1)
   - 15 g PHS-EVE (30 % EVE)
   - 85 g PGMEA as solvent

### Example 3:

### Preparation of patterned structures

Compositions A, B and C are used to prepare a patterned structure by photolithography.

In a first step, a coating of these compositions is applied on a bare silicon wafer (4 inch diameter) by means of a spin coater (2950 rpm). The coating is heat treated on a hot plate at 100°C for 1 minute and is subsequently exposed to electromagnetic radiation in an i-line stepper (35 mJ/cm²). After a post expose delay (PED) of 1 minute (or, alternatively, 4 minutes) the coating in the areas exposed to the radiation is removed by means of a 2.38 wt.-% aqueous tetramethylammonium hydroxide solution.

The thus obtained patterned structures are analyzed by CD-SEM as shown in fig. 1.

As can be seen in figure 1, a photoresist composition comprising a prior art compound (NIT) as the photoacid generator and that does not contain a quencher (Composition A) is characterized by a comparatively high sensitivity towards i-line radiation (as can be seen from the width of the uncoated area in the middle of the SEM-picture in fig. 1). However, increasing PED lead to a significant enlargement of the width of the uncoated area (from 5.84 µm to 7.14 µm), limiting the resolution of a patterned structure when using longer PED.

When using a photoresist composition comprising a prior art compound (NIT) as the photoacid generator and a separate quencher (triethylamine) (Composition B) the resolution at longer PED can be increased (as can be seen from the fact that the width of the uncoated area increases to a lower extend (i. e. from 5.21 to 5.55 µm) when changing the PED from 1 to 4 minutes). However, the sensitivity of such a composition is reduced compared to Composition B as can be seen from the fact that the width of the uncoated are is smaller, irrespective of the PED.

However, when using a photoresist composition comprising a photoacid generator according to the present invention (Composition C) the advantages of compositions A and B can be combined: the resolution at longer PED can be increased without scarifying the sensitivity of such a composition towards i-line radiation.

### EMBODIMENTS

**I** A compound, wherein the compound comprises at least one structural element A and at least one structural element B, wherein
   - structural element A is capable of releasing an acid upon exposure to electromagnetic radiation;
   - structural element B comprises at least one basic functional group, wherein the basic functional group comprises a primary, secondary or tertiary amine group.
**II** The compound according to embodiment I, wherein the compound is a non-ionic compound.
**III** The compound according to embodiment I or II, wherein the compound is selected from the group consisting of
   **ia)** a compound having the general formula (I) 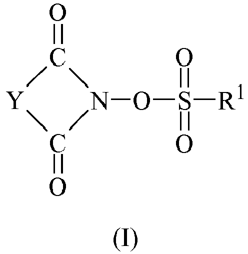 wherein Y and R¹ independently from each other represent an organic group and wherein Y carries the at least one basic functional group;
   **ib)** a compound having the general formula (II) 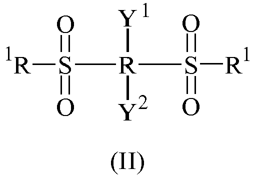 wherein Y¹, Y², R, R¹ and R² independently from each other represent an organic group and wherein at least one of Y¹ and Y² carries the at least one basic functional group;
   or
   ic) a compound having the general formula (IIIa) or (IIIb) 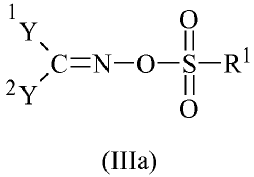 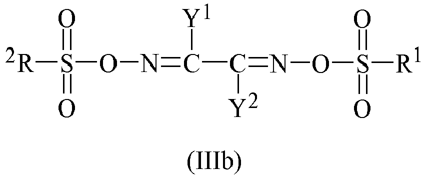 wherein Y¹, Y², R¹ and R² independently from each other represent an organic group and wherein at least one of Y¹ and Y² carries the at least one basic functional group.
IV The compound according to embodiment III, wherein R¹ is an optionally substituted, linear or branched aliphatic or heteroaliphatic group having 1-18 carbon atoms, or an aromatic or heteroaromatic group having 6-12 carbon atoms.
V The compound according to embodiment IV, wherein R¹ is a linear, branched or cyclic perfluoroalkyl group having the general formula -CₓF_{y}, in which x is an integer in the range from 1-8 and y is an integer in the range from 3-17.
VI The compound according to embodiment V, wherein R¹ is selected from the group consisting of -C₆F₅, -C₇F₇, -CF₃, -C₂F₅, -C₃F₇ and -C₄F₉.
VII The compound according to anyone of embodiments I to VI, wherein the at least one basic functional group has a pK_{A}-value in the range from 7.0 to 12.0.
VIII The compound according to anyone of embodiments I to VII, wherein the at least one basic functional group has the general formula (IVa) or (IVb) 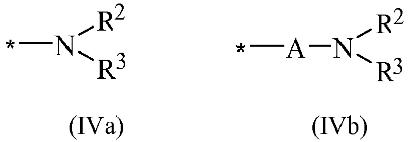 in which "*" indicates the bond to the compound and wherein
   - R² and R³ independently from each other represent a hydrogen atom, a linear or branched, optionally substituted aliphatic or heteroaliphatic group having 1-20 carbon atoms or an optionally substituted aromatic or heteroaromatic group having 6-20 carbon atoms,
      or
   - R² and R³ together form a heterocyclic group that, in addition to the nitrogen atom shown in the general formula (IVa) and (IVb), optionally comprises a further heteroatom selected from the group consisiting of N, O and S;
   - A represents an optionally substituted divalent aliphatic or heteroaliphatic group having 1-20 carbon atoms or a divalent aromatic or heteroaromatic group having 6-20 carbon atoms.
IX The compound according to embodiment VIII, wherein the at least one basic functional group has a general formula selected from the group consisting of formulae (Va) to (Vp): 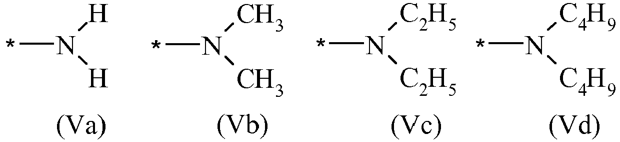 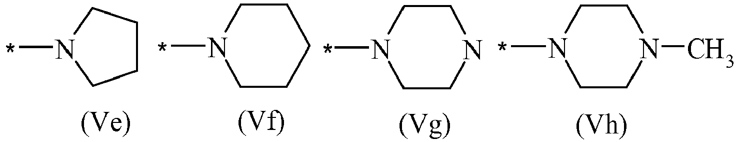 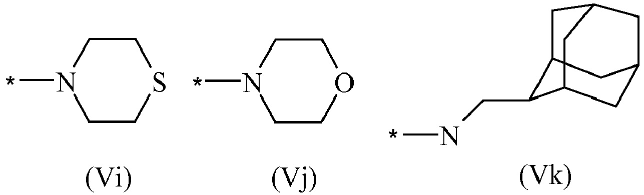 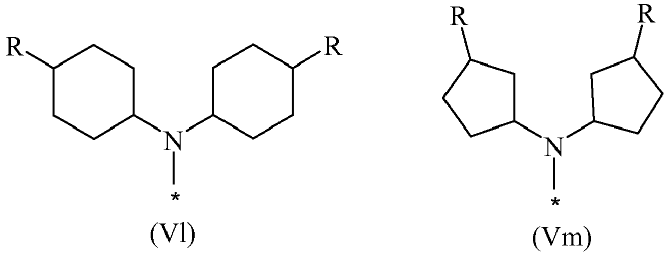 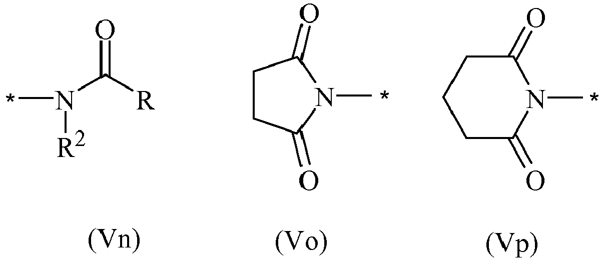 wherein R² is as defined in connection with the general formula (Iva) and (IVb) and R represents a hydrogen or a, aliphatic group having 1-20 carbon atoms.
X The compound according to anyone of embodiments I to IX, wherein the compound has the general formula (Ia) 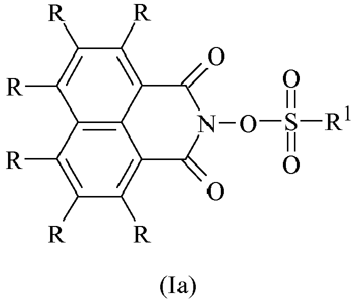 wherein R¹ is as defined in embodiments II to VI and wherein R independently from each other represents a hydrogen, a halogen, a hydroxyl group, a thiol group or an aliphatic or heteroaliphatic group with 1-20 carbon atoms, with the provisio that at least one of groups R represents a group -NR²R³ or -A-NR²R³ is as defined in embodiments VIII and IX.
XI The compound according to embodiment X, wherein the compound has the general formula (Ia-1), (Ia-2), (Ia-3) or (Ia-4): 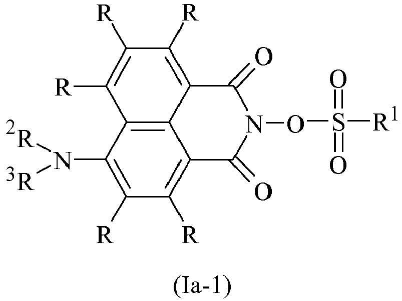 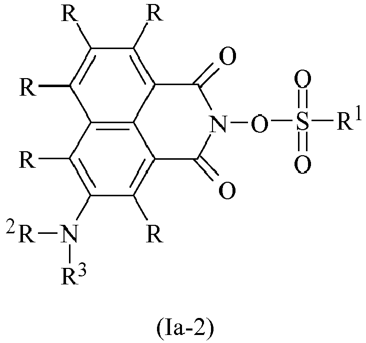 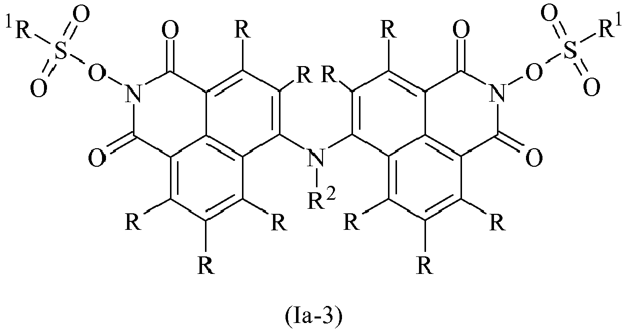 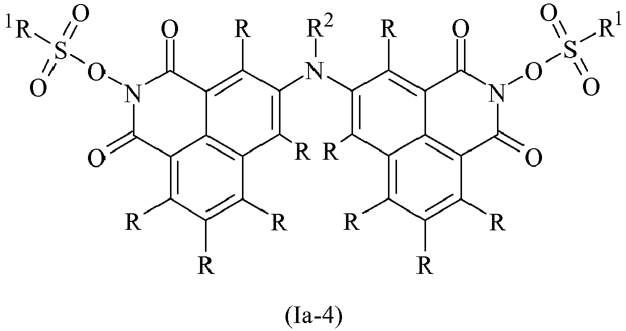 wherein R² and R³ are as defined in embodiments VIII and IX or wherein group -NR²R³ is as defined in embodiment X, R is defined as in embodiment X and R¹ is as defined in embodiments II to VI.
XII The compound according to embodiment X, wherein the compound has a general formula selected from the group consisting of formulae (Ia-a) to (Ia-r): 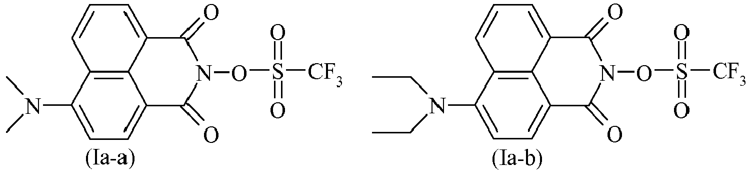 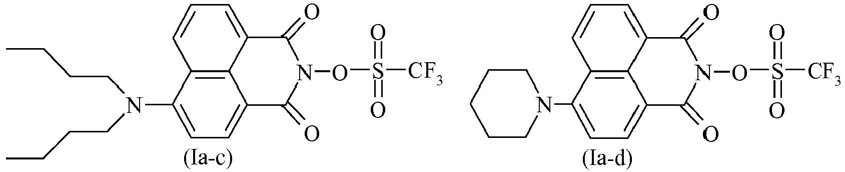 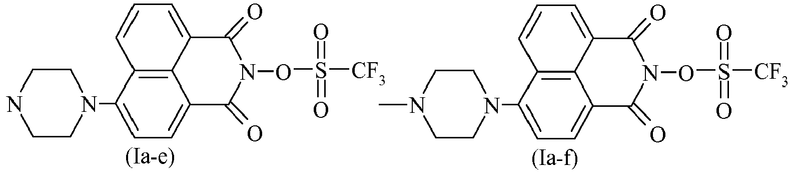 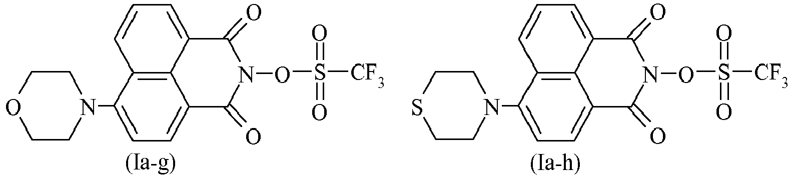 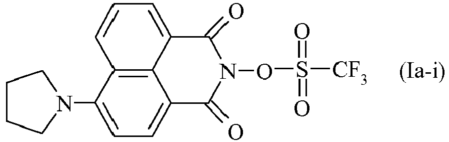 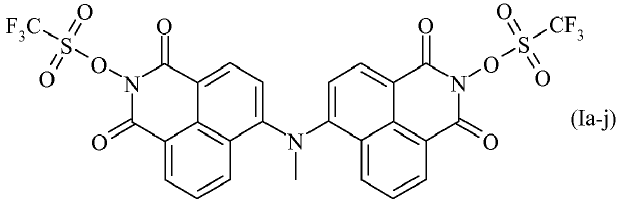 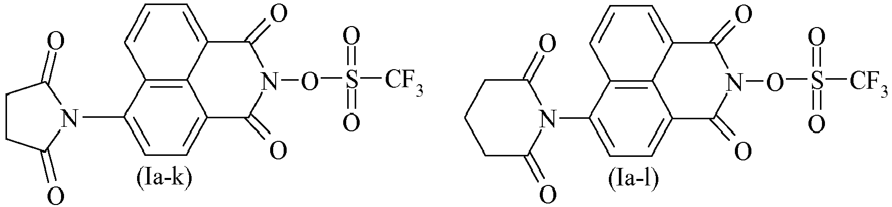 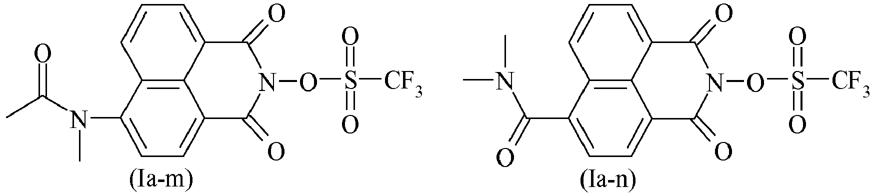 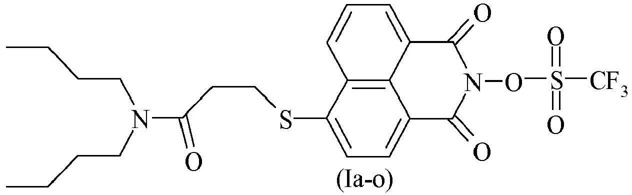 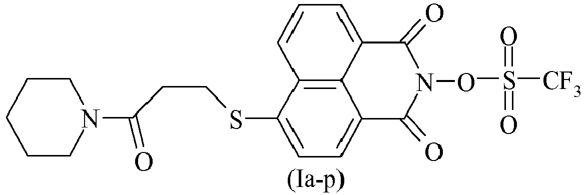 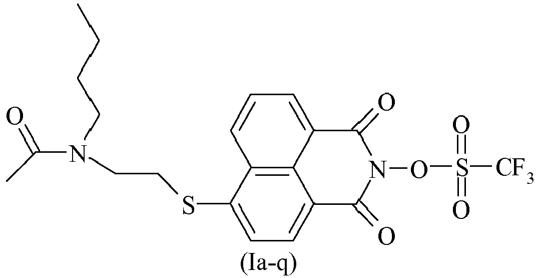 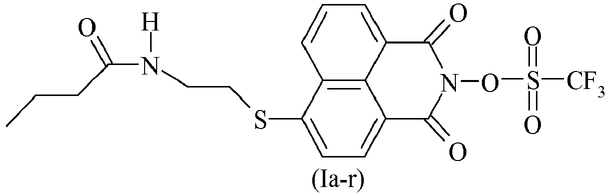
XIII A composition comprising
   (i) at least one compound according to anyone of embodiments I to XII;
   (ii) at least one compound capable of being imparted with an altered solubility in an aqueous solution in the presence of an acid;
   (iii) at least one organic solvent; and
   (iv) optionally at least one additive being different from components (i), (ii) and (iii).
XIV The composition according to embodiment XIII, wherein the at least one compound (ii) is a poly(hydroxystyrene)-resin in which at least a part of the hydroxy groups is substituted by protective groups.
XV The composition according to embodiment XIV, wherein the protective group is selected from the group consisting of a tert-butoxycarbonyloxy group, a tert-butyloxy group, a tert-amyloxycarbonyloxy group and an acetal group.
XVI The composition according to anyone of embodiments XIII to XV, wherein the at least one organic solvent (iii) is selected from the group consisting of a ketone, an ether and ester.
XVII The composition according to anyone of embodiments XIII to XVI, the composition comprising:
   (i) 0.05 to 15 wt.-% of a compound according to anyone of embodiments I to XII;
   (ii) 5 to 50 wt.-% of the compound capable of being imparted with an altered solubility in an aqueous solution in the presence of an acid;
   (iii) 0 to 10 wt.-% of the further additive; and
      wherein the reminder in the composition is the organic solvent.
XVIII A process for producing a composite comprising a substrate and a coating that is applied onto the substrate in a patterned structure, the process comprising the steps of
   (α1) applying a layer of the composition according to anyone of embodiments XIII to XVII onto the surface of the substrate and at least partial removal of the organic solvent (iv);
   (α2) exposing selected areas of the layer to electromagnetic radiation, thereby releasing an acid from the compound (i) in the areas exposed to the electromagnetic radiation;
   (α3) optionally heating the layer to impart compound (ii) in the areas in which the acid has been released with an altered solubility in an aqueous solution;
   (α4) at least partial removal of the layer.
XIX A composite obtained by the process according to embodiment XVIII.
XX A composite comprising a substrate and a coating applied on the surface of the substrate in a patterned structure, wherein the coating comprises a compound according to anyone of embodiments I to XII.
XXI The use of the compound according to anyone of embodiments I to XII for photoinduced polymerization, photoinduced crosslinking, photoinduced degradation and photoinduced transformation of functional groups.
XXII The use of the compound according to anyone of embodiments I to XII in protective coatings, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufacturing.

## Claims

1. A compound, wherein the compound comprises at least one structural element A and at least one structural element B, wherein
- structural element A is capable of releasing an acid upon exposure to electromagnetic radiation;
- structural element B comprises at least one basic functional group, wherein the basic functional group comprises a primary, secondary or tertiary amine group.

2. The compound according to claim 1, wherein the compound is a non-ionic compound.

3. The compound according to claim 1 or 2, wherein the compound is selected from the group consisting of
ia) a compound having the general formula (I) 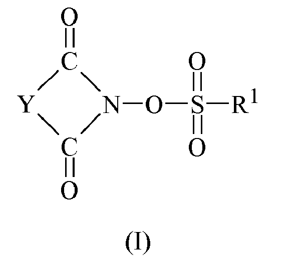 wherein Y and R¹ independently from each other represent an organic group and wherein Y carries the at least one basic functional group;
ib) a compound having the general formula (II) 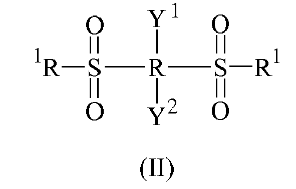 wherein Y¹, Y², R, R¹ and R² independently from each other represent an organic group and wherein at least one of Y¹ and Y² carries the at least one basic functional group;
or
ic) a compound having the general formula (IIIa) or (IIIb) 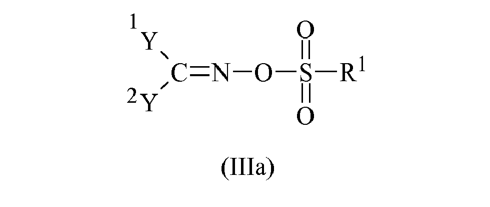 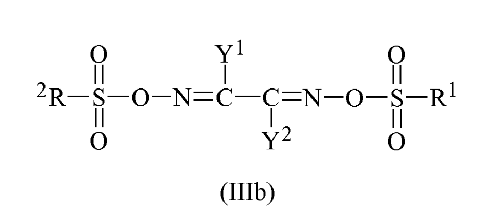 wherein Y¹, Y², R¹ and R² independently from each other represent an organic group and wherein at least one of Y¹ and Y² carries the at least one basic functional group.

4. The compound according to claim 3, wherein R¹ is an optionally substituted, linear or branched aliphatic or heteroaliphatic group having 1-18 carbon atoms, or an aromatic or heteroaromatic group having 6-12 carbon atoms.

5. The compound according to claim 4, wherein R¹ is a linear, branched or cyclic perfluoroalkyl group having the general formula -CₓF_{y}, in which x is an integer in the range from 1-8 and y is an integer in the range from 3-17.

6. The compound according to claim 5, wherein R¹ is selected from the group consisting of -C₆F₅, -C₇F₇, -CF₃, -C₂F₅, -C₃F₇ and -C₄F₉.

7. The compound according to anyone of claims 1 to 6, wherein the at least one basic functional group has a pK_{A}-value in the range from 7.0 to 12.0.

8. The compound according to anyone of claims 1 to 7, wherein the at least one basic functional group has the general formula (IVa) or (IVb) 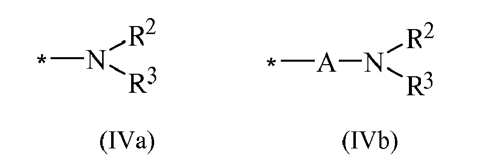 in which "*" indicates the bond to the compound and wherein
- R² and R³ independently from each other represent a hydrogen atom, a linear or branched, optionally substituted aliphatic or heteroaliphatic group having 1-20 carbon atoms or an optionally substituted aromatic or heteroaromatic group having 6-20 carbon atoms,
or
- R² and R³ together form a heterocyclic group that, in addition to the nitrogen atom shown in the general formula (IVa) and (IVb), optionally comprises a further heteroatom selected from the group consisiting of N, O and S;
- A represents an optionally substituted divalent aliphatic or heteroaliphatic group having 1-20 carbon atoms or a divalent aromatic or heteroaromatic group having 6-20 carbon atoms.

9. The compound according to claim 8, wherein the at least one basic functional group has a general formula selected from the group consisting of formulae (Va) to (Vp): 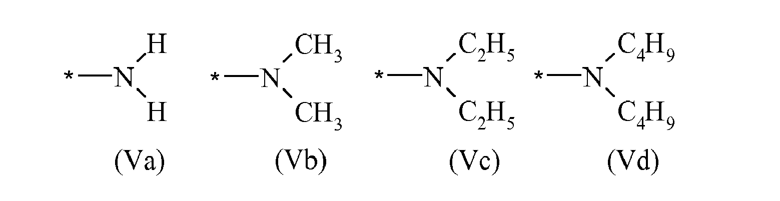 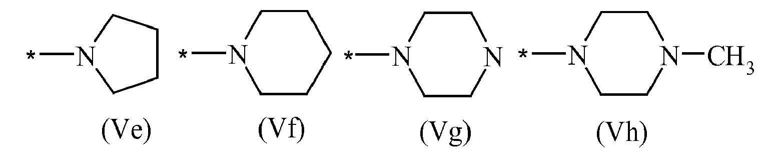 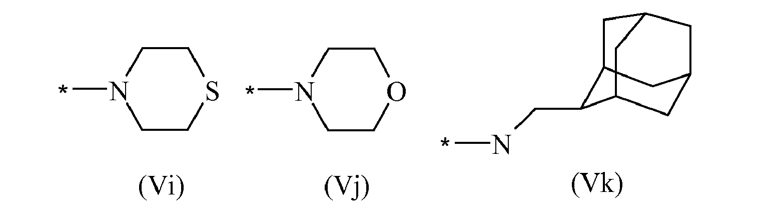 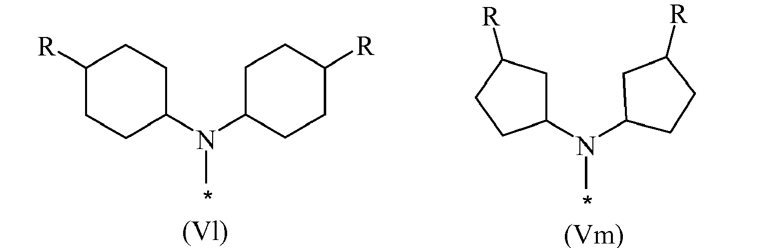 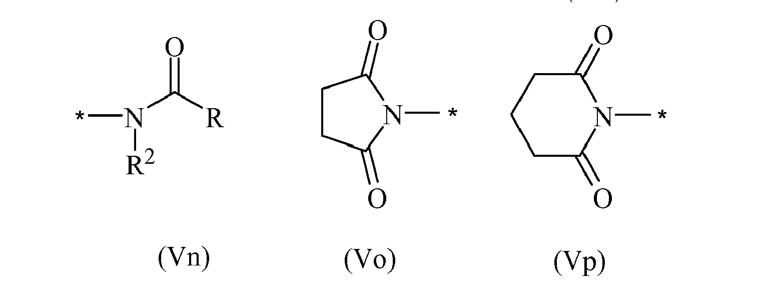 wherein R² is as defined in connection with the general formula (Iva) and (IVb) and R represents a hydrogen or a, aliphatic group having 1-20 carbon atoms.

10. The compound according to anyone of claims 1 to 9, wherein the compound has the general formula (Ia) 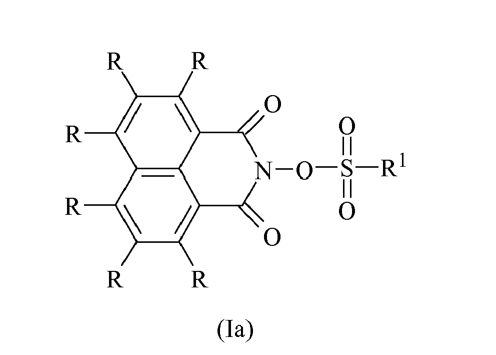 wherein R¹ is as defined in claims 2 to 6 and wherein R independently from each other represents a hydrogen, a halogen, a hydroxyl group, a thiol group or an aliphatic or heteroaliphatic group with 1-20 carbon atoms, with the provisio that at least one of groups R represents a group -NR²R³ or -A-NR²R³ is as defined in claims 8 and 9.

11. The compound according to claim 10, wherein the compound has the general formula (Ia-1), (Ia-2), (Ia-3) or (Ia-4): 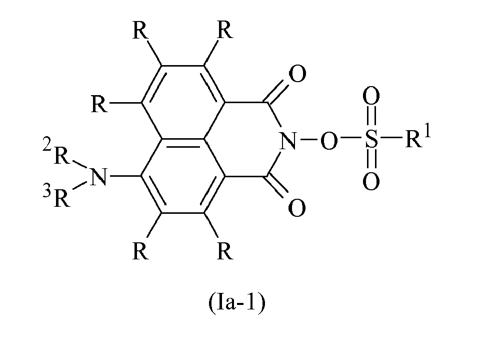 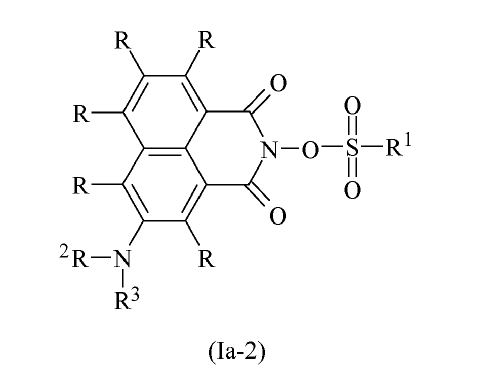 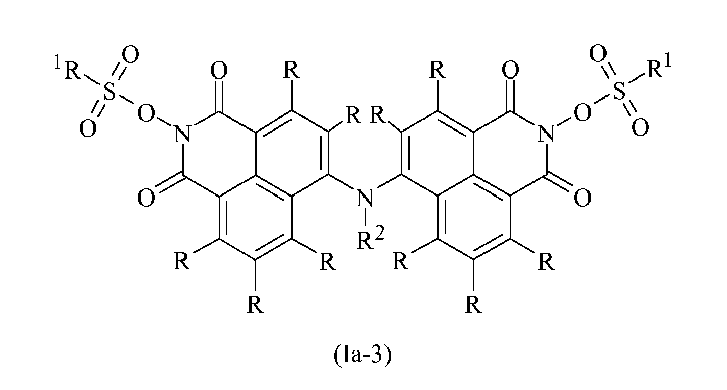 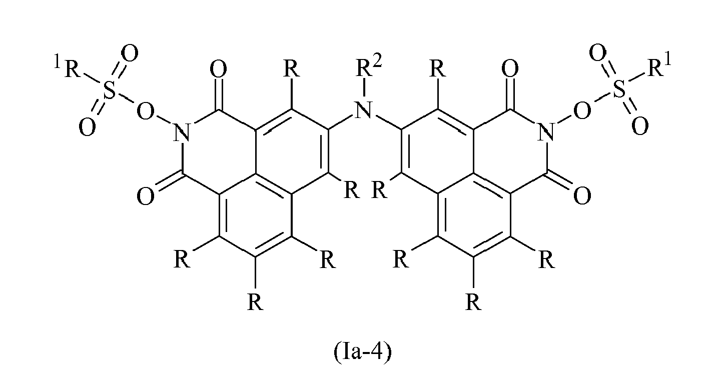 wherein R² and R³ are as defined in claims 8 and 9 or wherein group -NR²R³ is as defined in claim 10, R is defined as in claim 10 and R¹ is as defined in claims 2 to 6.

12. The compound according to claim 10, wherein the compound has a general formula selected from the group consisting of formulae (Ia-a) to (Ia-r):

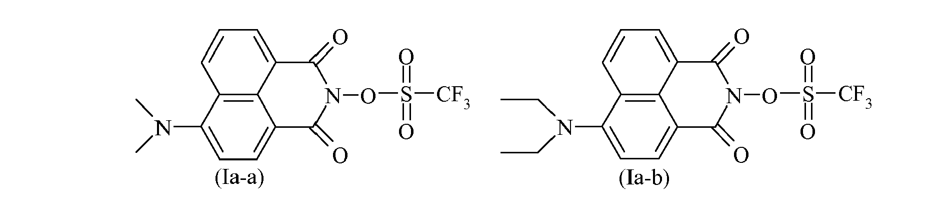

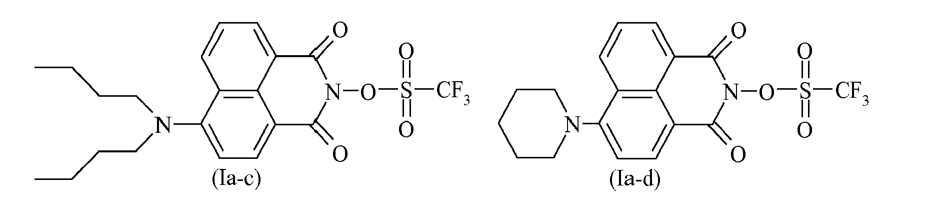

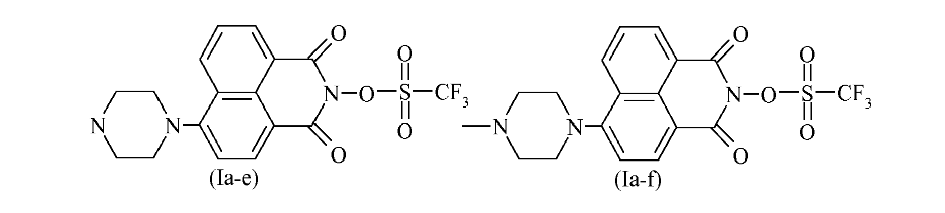

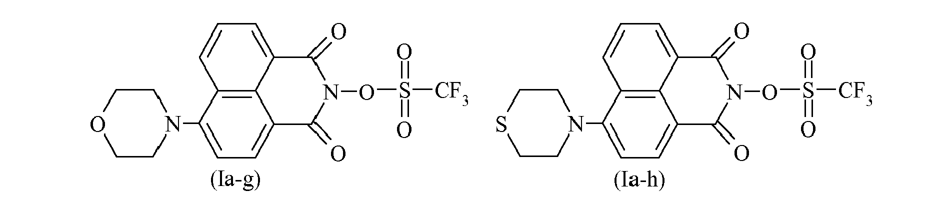

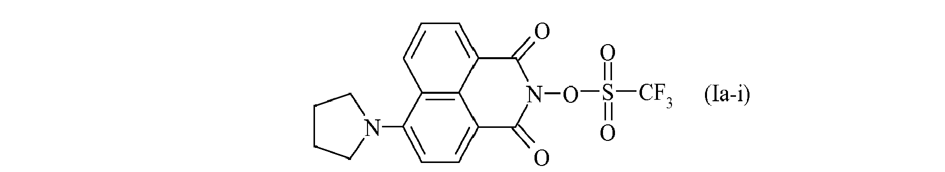

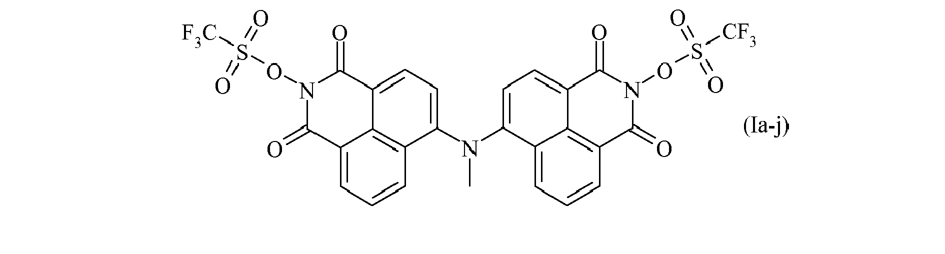

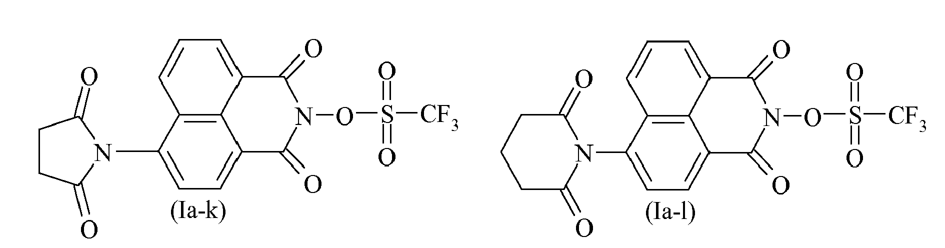

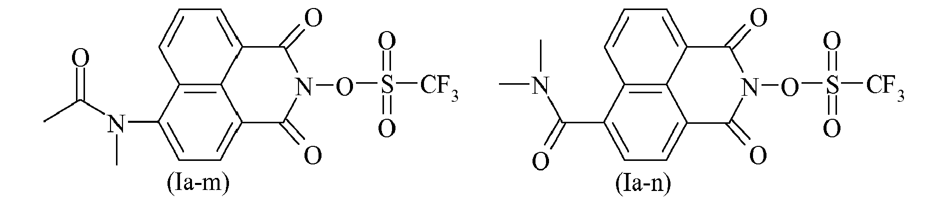

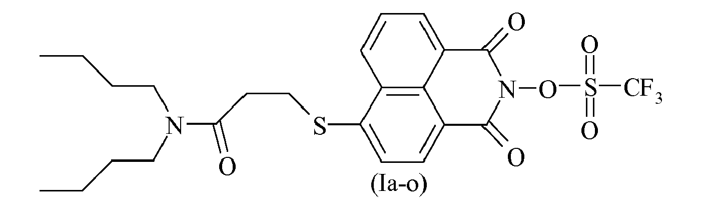

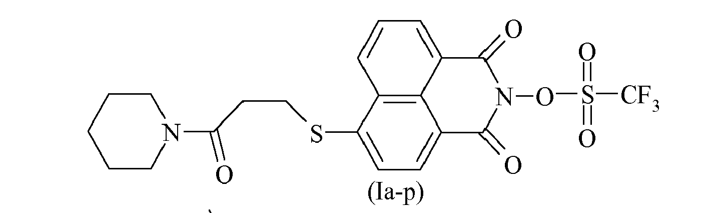

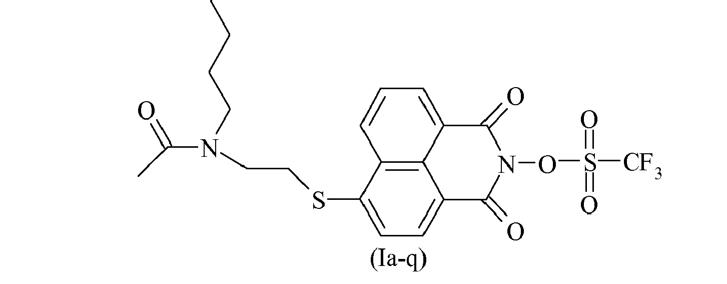

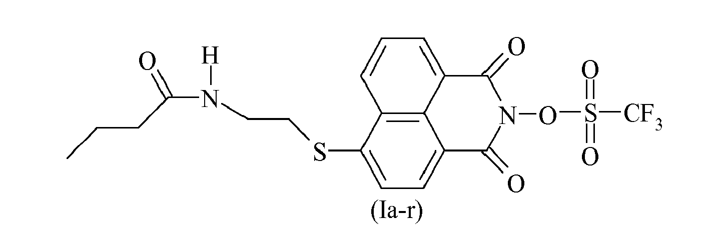

13. A composition comprising
(i) at least one compound according to anyone of claims 1 to 12;
(ii) at least one compound capable of being imparted with an altered solubility in an aqueous solution in the presence of an acid;
(iii) at least one organic solvent; and
(iv) optionally at least one additive being different from components (i), (ii) and (iii).

14. The composition according to claim 13, wherein the at least one compound (ii) is a poly(hydroxystyrene)-resin in which at least a part of the hydroxy groups is substituted by protective groups.

15. The composition according to claim 14, wherein the protective group is selected from the group consisting of a tert-butoxycarbonyloxy group, a tert-butyloxy group, a tert-amyloxycarbonyloxy group and an acetal group.

16. The composition according to anyone of claims 13 to 15, wherein the at least one organic solvent (iii) is selected from the group consisting of a ketone, an ether and ester.

17. The composition according to anyone of claims 13 to 16, the composition comprising:
(i) 0.05 to 15 wt.-% of a compound according to anyone of claims 1 to 12;
(ii) 5 to 50 wt.-% of the compound capable of being imparted with an altered solubility in an aqueous solution in the presence of an acid;
(iii) 0 to 10 wt.-% of the further additive; and
wherein the reminder in the composition is the organic solvent.

18. A process for producing a composite comprising a substrate and a coating that is applied onto the substrate in a patterned structure, the process comprising the steps of
(α1) applying a layer of the composition according to anyone of claims 13 to 17 onto the surface of the substrate and at least partial removal of the organic solvent (iv);
(α2) exposing selected areas of the layer to electromagnetic radiation, thereby releasing an acid from the compound (i) in the areas exposed to the electromagnetic radiation;
(α3) optionally heating the layer to impart compound (ii) in the areas in which the acid has been released with an altered solubility in an aqueous solution;
(α4) at least partial removal of the layer.

19. A composite obtained by the process according to claim 18.

20. A composite comprising a substrate and a coating applied on the surface of the substrate in a patterned structure, wherein the coating comprises a compound according to anyone of claims 1 to 12.

21. The use of the compound according to anyone of claims 1 to 12 for photoinduced polymerization, photoinduced crosslinking, photoinduced degradation and photoinduced transformation of functional groups.

22. The use of the compound according to anyone of claims 1 to 12 in protective coatings, smart cards, 3D rapid prototyping or additive manufacturing, sacrificial coatings, adhesives, antireflective coatings, holograms, galvano- and plating masks, ion implantation masks, etch resists, chemical amplified resists, light sensing applications, PCB(print circuit board) patterning, MEMS fabrication, TFT layer pattering on flat panel display, TFT layer pattering on flexible display, pixel pattering for display, in color filters or black matrix for LCD, or semiconductor patterning in packaging process and TSV related patterning on semiconductor manufacturing.
